# EUROPÄISCHE PATENTSCHRIFT

(11) **EP 1 247 503 B1**
(45) Veröffentlichungstag und Bekanntmachung des Hinweises auf die Patenterteilung: **28.09.2005**
(21) Anmeldenummer: 02005015.9
(22) Anmeldetag: 06.03.2002
(51) Int. Cl.: A61F 2/44, A61F 2/30

(54) **Implantat zum Einsetzen zwischen Wirbelkörper der Wirbelsäule**
Implant for insertion between two vertebral bodies of the spine
Implant pour poser entre deux vertèbres de la colonne vertébrale

(30) Priorität: 02.04.2001 DE 10116412
(43) Veröffentlichungstag der Anmeldung: 09.10.2002
(73) Patentinhaber: Ulrich GmbH & Co. KG, 89081 Ulm (DE)
(72) Erfinder: Sutcliffe, John, W1N 1 DL London (GB)
(74) Vertreter: Hentrich, Swen

(56) Entgegenhaltungen:
- EP-A- 0 560 140
- WO-A-01/15637
- WO-A-99/56675
- WO-A-99/63913
- DE-A- 4 109 941
- US-A- 5 916 267

## Beschreibung

Die Erfindung betrifft ein Implantat zum Einsetzen zwischen Wirbelkörper der Wirbelsäule als Platzhalter für aus der Wirbelsäule entfernte Wirbel, Wirbelteile oder Bandscheiben, bestehend aus zwei endständigen, zur Anlage an den angrenzenden Wirbel bestimmten Implantatteilen und einem dazwischen befindlichen mittigen Implantatteil.

Ein derartiges Implantat ist beispielsweise aus der DE 44 23 257 A1 bekannt, bei dem das mittige Implantatteil und die endständigen Implantteile hülsenförmig gestaltet und über Gewindeverbindungen miteinander gekoppelt sind. Dieses Implantat hat sich in der Praxis gut bewährt, da es gut zwischen den einander benachbarten Wirbeln positioniert werden kann und darüberhinaus aufgrund der Gewindeverbindungen eine einfache Distraktionsmöglichkeit während der Operation gegeben ist. Bei diesem Implantat hat es sich in der Praxis allerdings als wünschenswert gezeigt, eine stärkere frühzeitige Verankerung des Implantats zu erzielen, z.B. die Primär- und Rotationsstabilität zu erhöhen, ohne zusätzlich dorsale Stabilisierung oder ventrale Verplattung mit den bekannten Nachteilen wie hinsichtlich der Bauhöhe durchführen zu müssen.

Aus WO-A-99/56675 ist ein Implantat zum Ersatz von Wirbelkörpern bekannt, das die Merkmale des einleitenden Teils von Anspruch 1 aufweist.

Der Erfindung liegt daher die Aufgabe zugrunde, ein Implantat der eingangs genannten Art so auszubilden, daß während der Operation eine sofort belastbare und dauerhafte Verankerung des Implantats im Wirbelzwischenraum möglich ist.

Diese Aufgabe wird nach der Erfindung durch ein Implantat der eingangs genannten Art gelöst, das gekennzeichnet ist durch einen einseitig offenen Ring, der dem dem benachbarten Wirbel zugewandeten Ende von einem der endständigen Implantatteile zugeordnet ist und dessen Ringenden jeweils einen Durchgang zur Aufnahme einer Knochenschraube aufweisen,
wobei die freien Ringenden zum angrenzenden Wirbel abgekröpft und die abgekröpften Ringenden zur Bildung eines Schraubenkanals verdickt sind.

Dieses Implantat bietet den Vorteil, daß neben den aus dem Stand der Technik bekannten Verankerungen unter Verwendung beispielsweise von Verzahnungen an den Rändern der endständigen Implantatteile zusätzlich Knochenschrauben zum Einsatz kommen, die durch die Deckplatte in den Wirbel eingeschraubt werden, in den die Knochenschrauben tief eindrigen können und so zu einer großräumigen Verteilung der Belastung führen.

Die abgekröpften Ringenden bieten den Vorteil, daß die Knochenschraube geneigt in den Wirbelkörper hineingeschraubt werden kann und so besser sowohl axial als auch radial auftretende Belastungen aufnehmen kann
wobei die Verdickung auch eine höhere Belastbarkeit der Ringenden bewirkt.

Nach einer weiteren Ausführungsform der Erfindung ist vorgesehen, daß an dem anderen endständigen Implantatteil eine zu dessen benachbartem Wirbel weisende und diesen zumindest teilweise außenseitig überdeckende Fixierungsplatte mit mindestens einer Knochenschraubenaufnahme angeordnet ist. Diese Ausführungsform bietet dem Operateur weitere Gestaltungsmöglichkeiten bei der Verwendung des erfindungsgemäßen Implantats, bei dem gezielt auf die vorgegebenen knöchernen Strukturen eine Anpassung des Implantats erfolgen kann mit der Möglichkeit, eine Verankerung sowohl durch die Deckplatte als auch durch die Außenseiten der Wirbel zu bewirken. Die mit diesem Implantat verbundenen Vorteile zeigen sich besonders deutlich, wenn das Implantat in den Zwischenwirbelraum zwischen dem Lendenwirbel 5 und dem Os sacrum eingesetzt werden muß. Bei dem Lendenwirbel 5 kann ein endständiges Implantatteil verwendet werden, bei dem die einfachere Zugangsmöglichkeit für die Knochenschraube über die Fixierungsplatte ausgenutzt wird, während bei dem Os sacrum auf die Verwendung einer individuell anzupassenden Fixierungsplatte verzichtet ist, um die mit dem Ring verbundenen Vorteile auszunutzen, der ein tiefes, nicht oberflächennahes Eindringen der Knochenschraube ermöglicht.

Im Rahmen der Erfindung ist es allerdings auch möglich, daß an beiden endständigen Implantatteilen der einseitig offene Ring angeordnet ist, um so eine tiefe Verankerung in beiden Wirbeln zu ermöglichen und eine Behinderung der freien Sicht und der Zugänglichkeit zu der Kontaktstelle zwischen den endständigen Implantatteilen und den Deckplatten durch die Fixierungsplatte zu vermeiden.

Als zweckmäßig hat sich erwiesen, wenn der Schraubenkanal mit dem Ring einen Winkel der Größe von 25° bis 65° einschließt, wobei vorzugsweise der Winkel 45° beträgt.

Zur Vereinfachung der Handhabung des Implantats während der Operation ist vorgesehen, daß der Ring einstückig mit dem endständigen Implantatteil ausgebildet ist.

Ganz besonders bevorzugt ist eine Ausführungsform, die dadurch gekennzeichnet ist, daß das mittige Implantatteil hülsenförmig gestaltet ist und an seinen beiden Enden ein Gewinde aufweist zum Eingriff in ein Gewinde, das an hülsenförmigen Abschnitten der endständigen Implantatteile ausgebildet ist. Diese Ausführungsform bietet eine einfache Distraktionsmöglichkeit des Implantats, um so an die individuellen Gegebenheiten während der Operation eine Anpassung vornehmen zu können, die sich dann besonders einfach gestaltet, wenn eines der endständigen Implantatteile über ein Rechtsgewinde und das andere endständige Implantatteil über ein Linksgewinde mit dem mittigen Implantatteil verbunden ist, da so nur das mittige Implantatteil verdreht werden muß, um die Längenanpassung vorzunehmen.

Da die Wirbelsäule bei ihrem natürlichen Verlauf eine Lordose aufweist, liegen die Deckplatten benachbarter Wirbel nicht planparallel zueinander, sondern schließen einen Winkel mit ein, so daß der Zwischenwirbelraum eine keilförmige Gestalt hat. Daher ist es günstig, wenn bei mindestens einem der endständigen Implantatteile der Ring geneigt zur Gewindeachse verläuft, um eine Stabilisierung der Wirbelsäule in der ihrem natürlichen Verlauf entsprechenden Konfiguration zu erreichen.

Für eine provisorische Fixierung des Implantats vor dem Einbringen der Knochenschrauben ist es hilfreich, wenn an den endständigen Implantatteilen an den der Grundplatte des benachbarten Wirbel zur Anlage kommenden Fläche Zähne ausgebildet sind. Günstig ist es auch, wenn das Implantatinnere über Öffnungen mit dem Außenraum verbunden ist, da so Knochenzement und/oder autologes oder homologes Knochenmaterial oder ein anderes biokompatibles, bioresorbierbares oder biologisches Material in das Implantatinnere verbracht werden kann und zudem das Einwachsen des Implantats gefördert ist, das zweckmäßigerweise aus Titan besteht, das leicht und hoch belastbar ist und darüberhinaus eine Kontrolle der Lage des Implantats durch Röntgenuntersuchungen ermöglicht. Als alternative Werkstoffe kommen ein biokompatibler Kunststoff, insbesondere Poly-Ether-Ether-Keton (PEEK) und/oder Biokeramik und/oder Knochen in Frage.

Im folgenden wird die Erfindung an in der Zeichnung dargestellten Ausführungsbeispielen näher erläutert; es zeigen:
- Fig. 1: eine Seitenansicht des erfindungsgemäßen Implantats, teilweise geschnitten dargestellt,
- Fig. 2: eine Vorderansicht des den einseitig offenen Ring aufweisenden Implantatteils,
- Fig. 3: eine Draufsicht auf das in Fig. 2 gezeigte endständige Implantatteil, durch Knochenschrauben verankert auf der Deckplatte eines Wirbels,
- Fig. 4: eine Seitenansicht des im Zwischenwirbelraum zwischen zwei Wirbeln verankerten Implantats aus Fig. 1,

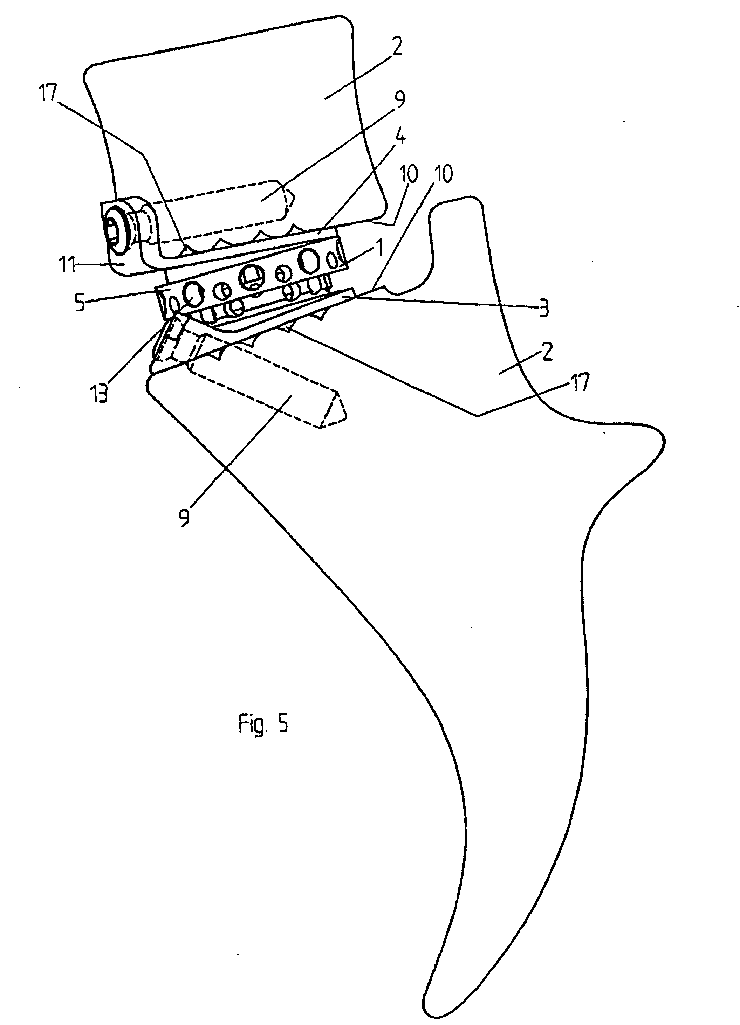

- Fig. 5: eine der Fig. 4 entsprechende Darstellung des im Zwischenwirbelraum zwischen dem Lendenwirbel 5 und dem Os sacrum angeordneten Implantats, und

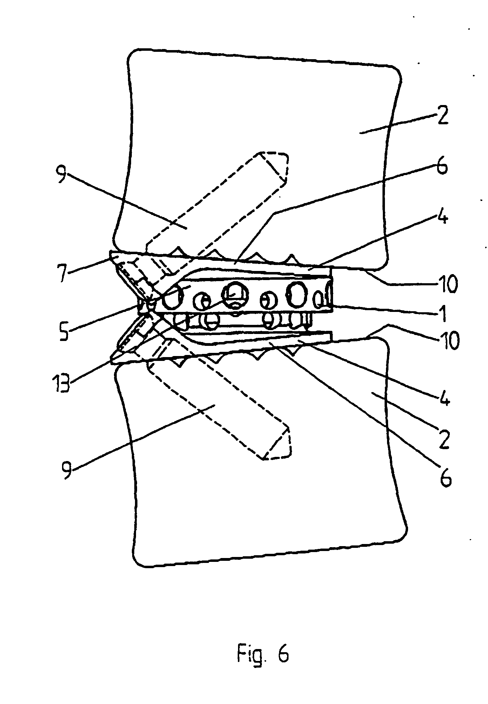

- Fig. 6: eine der Fig. 4 entsprechende Darstellung eines Implantats mit den beiden jeweils einen einseitig offenen Ring aufweisenden endständigen Implantatteilen.

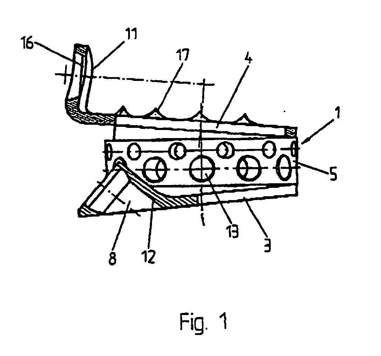

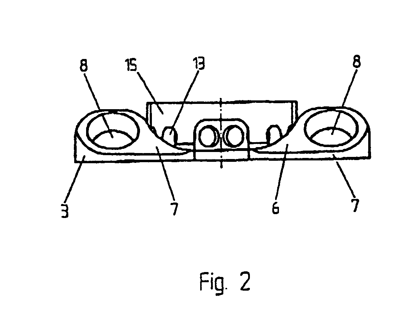

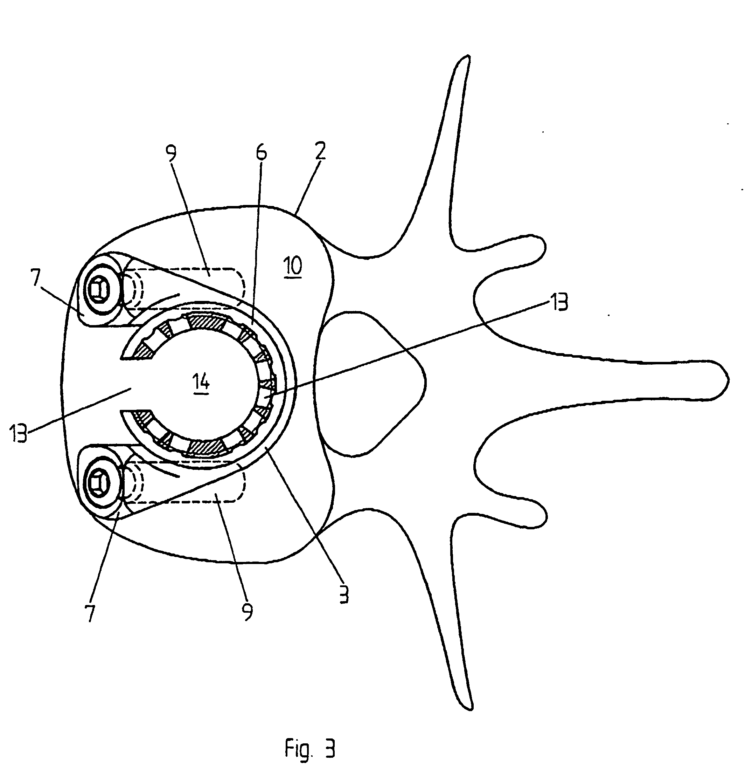

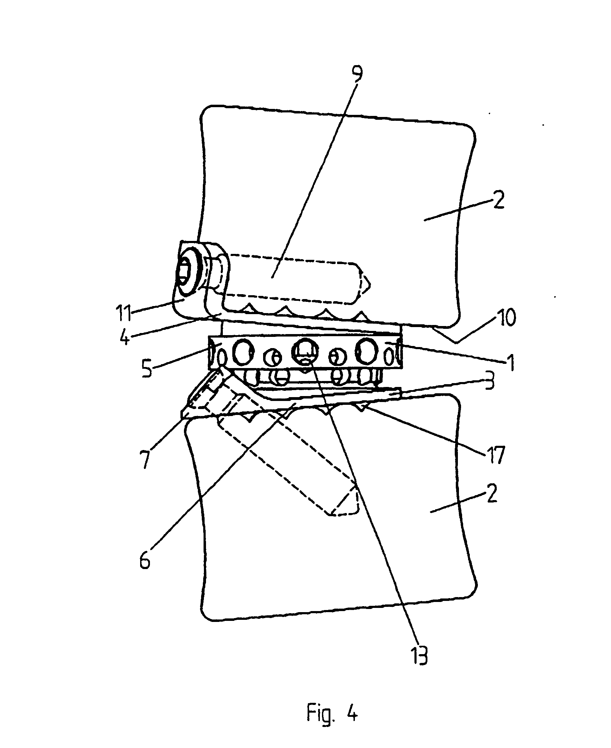

Das in der Zeichnung dargestellte Implantat 1 dient zum Einsetzen zwischen Wirbelkörper 2 der Wirbelsäule als Platzhalter für aus der Wirbelsäule entfernte Wirbel, Wirbelteile oder Bandscheiben. Das Implantat 1 besteht aus zwei endständigen, zur Anlage an den angrenzenden Wirbeln bestimmten Implantatteilen 3, 4 und einem dazwischen befindlichen mittigen Implantatteil 5, wobei das mittige Implantatteil 5 hülsenförmig gestaltet ist und an seinen beiden Enden ein Gewinde aufweist zum Eingriff in ein Gewinde, das an koaxialen hülsenförmigen Abschnitten 15 der endständigen Implantatteile 3, 4 ausgebildet ist. Dabei ist eines der endständigen Implantatteile 3 über ein Rechtsgewinde und das andere endständige Implantatteil 4 über ein Linksgewinde mit dem mittigen Implantatteil 5 verbunden. Bei dem in Fig. 1 dargestellten Implantat 1 besitzt das untere endständige Implantatteil 3 einen einseitig offenen Ring 6, der dem dem benachbarten Wirbel zugewandten Ende des endständigen Implantatteils 3 zugeordnet ist. Wie insbesondere aus den Fig. 2 und 3 erkenntlich, weisen die freien Ringenden 7 jeweils einen Durchgang 8 auf, durch die hindurch Knochenschrauben 9 in die Deckplatte 10 des Wirbels eingebracht werden können. Das in Fig. 1 dargestellte Implantat 1 besitzt weiterhin an dem anderen oberen endständigen Implantatteil 4 eine zu dessen benachbartem Wirbel weisende und diesen zumindest teilweise außenseitig überdeckende Fixierungsplatte 11 mit zumindest einer Knochenschraubenaufnahme 16, wobei es allerdings gemäß dem in Fig. 4 dargestellten Ausführungsbeispiel gleichfalls möglich ist, das an beiden endständigen Implantatteilen 3, 4 der einseitig offene Ring 6 angeordnet ist.

Die freien Ringenden 7 des einseitig offenen Ringes 6 sind zum gegenüberliegenden Wirbel abgekröpft und zur Bildung eines Schraubenkanals 12 verdickt, der mit dem Ring 6 einen Winkel der Größe von 25° bis 65°, in dem gezeigten Ausführungsbeispiel von 45° einschließt.

Der Ring 6 ist einstückig mit dem endständigen Implantatteil 3, 4 ausgebildet.

Aus den in der Zeichnung dargestellten Figurenbeispielen ist weiterhin ersichtlich, daß der Ring 6 gegenüber den das Gewinde tragenden hülsenförmigen Abschnitten der endständigen Implantatteile 3, 4 geneigt zur Gewindeachse verläuft. Das Implantat 1, das aus biokompatiblen, biologischen oder bioresorbierbaren Material besteht, wofür insbesondere chirurgischer Stahl, Titan, Knochen, Biokeramik oder PEEK in Frage kommen, weist Öffnungen 13 auf, über die das Implantatinnere 14 mit dem Außenraum verbunden ist, so daß auch in das Implantat 1 Knochen hineinwachsen kann, wobei im übrigen durch den einseitig offenen Ring 6 ein sich von Wirbel zu Wirbel erstreckender, durchgehender Zugang vorliegt, in dem sich eine vollständige Knochenstraße ausbilden kann und weiterhin eine gute Kontrollmöglichkeit auch mittels Röntgenaufnahmen besteht. Die endständigen Implantatteile 3, 4 weisen an den an der Grundplatte 10 zur Anlage kommenden Flächen Zähne 17 auf.

## Patentansprüche

1. Implantat zum Einsetzen zwischen Wirbelkörper (2) der Wirbelsäule als Platzhalter für aus der Wirbelsäule entfernte Wirbel, Wirbelteile oder Bandscheiben, bestehend aus zwei endständigen, zur Anlage an den angrenzenden Wirbel bestimmten Implantatteilen (3, 4) und einem dazwischen befindlichen mittigen Implantatteil (5), **gekennzeichnet durch** einen einseitig offenen Ring (6), der dem dem benachbarten Wirbel zugewandten Ende von einem der endständigen Implantatteile (3, 4) zugeordnet ist, wobei die freien Ringenden (7) des einseitig offenen Ringes (6) jeweils einen Durchgang (8) zur Aufnahme einer Knochenschraube (9) aufweisen, die in die zum benachbarten Wirbel gehörende Deckplatte (10) einzubringen ist, und wobei die freien Ringenden (7) zum angrenzenden Wirbel abgekröpft und die abgekröpften Ringenden (7) zur Bildung eines Schraubenkanals (12) verdickt sind.

2. Implantat nach Anspruch 1, **dadurch gekennzeichnet, daß** an dem anderen endständigen Implantatteil (3, 4) eine zu dessen benachbartem Wirbel weisende und diesen zumindest teilweise außenseitig überdeckende Fixierungsplatte (11) mit mindestens einer Knochenschraubenaufnahme (16) angeordnet ist.

3. Implantat nach Anspruch 1, **dadurch gekennzeichnet, daß** an beiden endständigen Implantatteilen (3, 4) der einseitig offene Ring (6) angeordnet ist.

4. Implantat nach einem der Ansprüch 1 bis 3, **dadurch gekennzeichnet, daß** der Schraubenkanal (12) mit dem Ring (6) einen Winkel der Größe von 25° bis 65° einschließt.

5. Implantat nach Anspruch 4, **dadurch gekennzeichnet, daß** der Winkel 45° beträgt.

6. Implantat nach einem der Ansprüche 1 bis 5, **dadurch gekennzeichnet, daß** der Ring (6) einstückig mit dem endständigen Implantatteil (3, 4) ausgebildet ist.

7. Implantat nach einem der Ansprüche 1 bis 6, **dadurch gekennzeichnet, daß** das mittige Implantatteil (5) hülsenförmig gestaltet ist und an seinen beiden Enden ein Gewinde aufweist zum Eingriff in ein Gewinde, das an hülsenförmigen Abschnitten (15) der endständigen Implantatteile (3, 4) ausgebildet ist.

8. Implantat nach Anspruch 7, **dadurch gekennzeichnet, daß** eines der endständigen Implantatteile (3) über ein Rechtsgewinde und das andere endständige Implantatteil (4) über ein Linksgewinde mit dem mittigen Implantatteil (5) verbunden ist.

9. Implantat nach Anspruch 7 oder 8, **dadurch gekennzeichnet, daß** bei mindestens einem der endständigen Implantatteile (3, 4) der Ring (6) geneigt zur Gewindeachse verläuft.

10. Implantat nach einem der Ansprüche 1 bis 9, **dadurch gekennzeichnet**, das an den endständigen Implantatteilen (3, 4) an den der Grundplatte (10) des benachbarten Wirbel zur Anlage kommenden Fläche Zähne (17) ausgebildet sind.

11. Implantat nach einem der Ansprüche 1 bis 10, **dadurch gekennzeichnet, daß** das Implantatinnere (14) über Öffnungen (13) mit dem Außenraum verbunden ist.

12. Implantat nach einem der Ansprüche 1 bis 11, **dadurch gekennzeichnet, daß** es aus Titan besteht.

13. Implantat nach einem der Ansprüche 1 bis 12, **dadurch gekennzeichnet, daß** als Werkstoff ein biokompatibler Kunststoff, insbesondere Poly-Ether-Ether-Keton (PEEK) und/oder Biokeramik und/oder Knochen genutzt ist.

## Claims

1. Implant for insertion between vertebral bodies (2) of the spinal column as replacement for the vertebrae, vertebra parts or intervertebral discs removed from the spinal column comprising two terminal implant parts (3, 4) specified for insertion against the bordering vertebra and a middle implant part (5) situated between them, **characterized by** a ring, (6) open on one side, which is allocated to that end of one of the terminal implant parts (3, 4) which faces the adjacent vertebra, the free ring-ends (7) of the ring (6) open on one side each having a passage (8) for housing a bone screw (9) which is to be introduced into the roof plate (10) belonging to the adjacent vertebra, and the free ring-ends (7) bent at a right angle vis-à-vis the adjacent vertebra and the ring-ends (7) bent at a right angle being thickened to form a screw channel (12).

2. Implant according to claim 1, **characterized in that** there is arranged at the other terminal implant part (3, 4) a fixing plate (11) pointing to its adjacent vertebra and at least partly covering this on the outside, with at least one bone screw housing (16).

3. Implant according to claim 1, **characterized in that** the ring (6) open on one side is arranged at both terminal implant parts (3, 4).

4. Implant according to one of claims 1 to 3, **characterized in that** the screw channel (12) forms an angle of the order of 25° to 65° with the ring (6).

5. Implant according to claim 4, **characterized in that** the angle is 45°.

6. Implant according to one of claims 1 to 5, **characterized in that** the ring (6) is developed in one piece with the terminal implant part (3, 4) [TN].

7. Implant according to one of claims 1 to 6, **characterized in that** the middle implant part (5) is sleeve-shaped and has at its two ends a thread for engagement in a thread which is developed at the sleeve-shaped sections (15) of the terminal implant parts (3, 4).

8. Implant according to claim 7, **characterized in that** one of the terminal implant parts (3) is connected to the middle implant part (5) via a right-handed thread and the other terminal implant part (4) via a left-handed thread.

9. Implant according to claim 7 or 8, **characterized in that** the ring (6) runs inclined to the thread axis in the case of at least one of the terminal implant parts (3, 4).

10. Implant according to one of claims 1 to 9, **characterized in that** teeth (17) are developed at the terminal implant parts (3, 4) at the surfaces abutting the roof plate (10) of the adjacent vertebra.

11. Implant according to one of claims 1 to 10, **characterized in that** the inside of the implant (14) is connected to the outside via openings (13).

12. Implant according to one of claims 1 to 11, **characterized in that** it consists of titanium.

13. Implant according to one of claims 1 to 12, **characterized in that** a biocompatible plastic, in particular polyetheretherketone (PEEK) and/or bioceramics and/or bone is used as material.

## Revendications

1. Implant destiné à être posé entre des vertèbres (2) de la colonne vertébrale, en tant qu'élément de substitution de vertèbres, de parties de vertèbres ou de disques intervertébraux qui ont été retirés de la colonne vertébrale, constitué de deux éléments d'extrémité d'implant (3, 4), destinés à être appliqués contre la vertèbre contiguë, et d'un élément médian d'implant (5) disposé entre les deux éléments d'extrémité, **caractérisé par** une bague (6) qui est ouverte sur un côté et est associée à l'extrémité de l'un des éléments d'extrémité d'implant (3, 4) tournée vers la vertèbre voisine, les extrémités libres (7) de la bague (6) ouverte sur un côté présentant chacune un passage (8) pour recevoir une vis à os (9) qui doit être insérée dans la plaque de couverture (10) faisant partie de la vertèbre voisine, et les extrémités de bague (7) libres étant coudées en direction de la vertèbre contiguë, et les extrémités de bague (7) coudées étant plus épaisses aux fins de former un conduit à vis (12).

2. Implant selon la revendication 1, **caractérisé par le fait qu'**une plaque de fixation (11), comportant au moins un logement (16) de vis à os, est disposée sur l'autre élément d'extrémité d'implant (3, 4) et est orientée en direction de la vertèbre contiguë à cet élément, en recouvrant au moins partiellement cette vertèbre sur l'extérieur.

3. Implant selon la revendication 1, **caractérisé par le fait que** la bague (6) ouverte sur un côté est disposée sur les deux éléments d'extrémité d'implant (3, 4).

4. Implant selon une des revendications 1 à 3, **caractérisé par le fait que** le conduit à vis (12) forme avec la bague (6) un angle dont la valeur est comprise entre 25° et 65°.

5. Implant selon la revendication 4, **caractérisé par le fait que** l'angle est de 45°.

6. Implant selon une des revendications 1 à 5, **caractérisé par le fait que** la bague (6) est réalisée d'une seule pièce avec l'élément d'extrémité d'implant (3, 4).

7. Implant selon une des revendications 1 à 6, **caractérisé par le fait que** l'élément médian d'implant (5) est conformé en manchon et présente à ses deux extrémités un filet destiné à entrer en prise avec un filet qui est aménagé sur des tronçons (15) en forme de manchons des éléments d'extrémité d'implant (3, 4).

8. Implant selon la revendication 7, **caractérisé par le fait que** l'un des éléments d'extrémité d'implant (3) est relié à l'élément médian d'implant (5) par l'intermédiaire d'un filet à droite et l'autre élément d'extrémité d'implant (4) est relié à l'élément médian (5) par un filet à gauche.

9. Implant selon la revendication 7 ou 8, **caractérisé par le fait que** sur au moins un des éléments d'extrémité d'implant (3, 4) la bague (6) est inclinée par rapport à l'axe de filet.

10. Implant selon une des revendications 1 à 9, **caractérisé par le fait que** des dents (17) sont formées sur les éléments d'extrémité d'implant (3, 4), sur les surfaces venant en appui contre la plaque de base (10) de la vertèbre voisine.

11. Implant selon une des revendications 1 à 10, **caractérisé par le fait que** l'intérieur (14) de l'implant communique avec l'extérieur via des ouvertures (13).

12. Implant selon une des revendications 1 à 11, **caractérisé par le fait qu'**il est en titane.

13. Implant selon une des revendications 1 à 12, **caractérisé par le fait que** le matériau utilisé est une matière plastique biocompatible, en particulier du polyéther-éther-cétone (PEEK) et/ou une biocéramique et/ou de l'os.
